# EUROPÄISCHE PATENTSCHRIFT

(11) **EP 2 000 458 B1**
(45) Veröffentlichungstag und Bekanntmachung des Hinweises auf die Patenterteilung: **04.05.2011**
(21) Anmeldenummer: 08102430.9
(22) Anmeldetag: 10.03.2008
(51) Int. Cl.: C07C 209/62

(54) **Verfahren zur Herstellung von Aminen**
Method for manufacturing amines
Procédé de fabrication d'amines

(30) Priorität: 10.05.2007 DE 102007022445
(43) Veröffentlichungstag der Anmeldung: 10.12.2008
(73) Patentinhaber: Evonik Degussa GmbH, 45128 Essen (DE)
(72) Erfinder: Köhler, Günther, Dr., 45657 Recklinghausen (DE); Neumann, Manfred, Dr., Marl 45770 (DE); Omeis, Marianne, Dr., Dorsten 46286 (DE)

(56) Entgegenhaltungen:
- EP-A- 0 480 493
- EP-A- 1 195 194
- EP-A- 1 700 847
- EWING ET AL.: "Mechanism of Cleavage of Carbonate Anions." J. AM. CHEM. SOC., Bd. 102, Nr. 9, 23. April 1980 (1980-04-23), Seiten 3072-3084, XP002502326
- JACQUEMARD U ET AL: "Mild and selective deprotection of carbamates with Bu4NF" TETRAHEDRON, ELSEVIER SCIENCE PUBLISHERS, AMSTERDAM, NL, Bd. 60, Nr. 44, 25. Oktober 2004 (2004-10-25), Seiten 10039-10047, XP004580207 ISSN: 0040-4020

## Beschreibung

Die vorliegende Erfindung beschreibt ein Verfahren zur Herstellung von Aminen durch katalytische Spaltung von Carbamaten.

Die Spaltung beziehungsweise die Decarboxylierung von Carbamaten ist ein gebräuchliches Verfahren zur Herstellung von Aminen. Die erhaltenen Amine sind wiederum Ausgangsverbindungen für zahlreiche technische Zwischenprodukte und Pharmawirkstoffe.

Den allgemeinen Reaktionsmechanismus der Spaltung von Carbamaten beschreiben Ewing et al. in ihrer Veröffentlichung J. Amer. Chem. Soc. (1980) 102(9), 3072-3084:

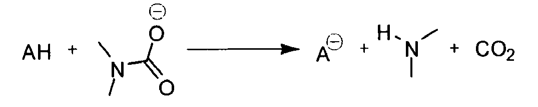

Die Spaltung von Carbamaten in Gegenwart von Tetra-n-butylammoniumfluorid (Bu₄NF) beschreiben Jacquemard et al. in Tetrahedron (2004) 60(44), 10039 - 10047: 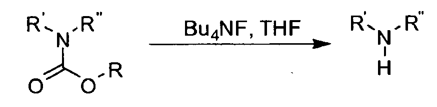 mit
R = Methyl, Ethyl, tert.-Butyl, Benzyl, Allyl oder Phenyl,
R' = Alkyl oder Aryl
R" = Wasserstoff, Alkyl oder Aryl.

Die allgemeine Struktur der Carbamate beschreibt Gaines in J. Org. Chem. (1985) 50, 410 - 411 als monomolekulares Zwitterion oder Di-Salz.

Die japanische Veröffentlichung JP 2006-069941 A beschreibt u.a. die thermische Zersetzung von Carbamaten zur Herstellung von Isocyanaten. Hingegen beschreibt die JP 2004-262892 A die thermische Zersetzung von Carbamaten in Gegenwart eines Zinn-Katalysators und eines Feststoffkatalysator, ausgewählt aus Silikaten, Kieselgel und/oder Metall.

Die Zersetzung von Ammoniumcarbamat in Ammoniak und Kohlendioxid beschreiben die WO 2006/094541 A und die EP 1 195 194 A.

Die Spaltung von Carbamaten in Gegenwart eines Zinnkatalysators wird auch in JP 2004-262892 A beschrieben. Der Einsatz von Jod zur Spaltung von Carbamaten beschreiben Vatele et al. in Tetrahedron Letters (2003), 44(51), 9127-9129.

Es war die Aufgabe der vorliegenden Erfindung, ein Verfahren zur Spaltung von Carbamaten zur Verfügung zu stellen, das es ermöglicht, bei niedrigen Temperaturen unter Entfernung des Kohlendioxids das Carbamat zu spalten. Vor allem sollte vermieden werden, dass sich das Carbamat aus den Reaktionsprodukten Kohlendioxid und gewünschten Amin zurückbildet. Insbesondere war es die Aufgabe ein kontinuierliches Herstellverfahren hierfür zu entwickeln.

Überraschenderweise konnte gefunden werden, dass ein Verfahren zur Herstellung von Aminen durch Spaltung von Carbamaten in Gegenwart einer Säure eine kontinuierliche Verfahrensweise ermöglicht. Bei den in dem erfindungsgemäßen Verfahren eingesetzten Säuren handelt es sich um hochsiedende Neosäuren, die überraschenderweise über eine katalytische Wirkung bei der Carbamatspaltung verfügen. Neben der katalytischen Wirkung dienen diese Säuren in dem erfindungsgemäßen Verfahren auch als Sumpfverdünner. Umso mehr war es überraschend, dass es gelang, das zu spaltende Carbamat als Lösung oder auch als Suspension einzusetzen, wobei Kohlendioxid und das eingesetzte Lösemittel des Carbamats aus der Reaktionszone entfernt werden konnten, ohne dass das Zielprodukt Amin mit entfernt wird. Aufgrund der niedrigeren Reaktionstemperatur, die durch den Einsatz der Säuren ermöglicht wird, verbleibt das Zielprodukt Amin in der Säure, die hiermit neben Katalysator auch als Lösemittel für das Amin dient. Auf diese Weise ermöglicht das erfindungsgemäße Verfahren die separate Abtrennung des entstehenden Kohlendioxids und des Zielprodukts Amin. Die Rückbildung des Carbamats kann somit verhindert werden. Dies ist ein entscheidender Vorteil gegenüber den Verfahren gemäß dem Stand der Technik, da hier die Spaltung des Carbamats bei höheren Temperaturen verläuft, so dass sich sowohl das Kohlendioxid als auch das Zielprodukt Amin in die Gasphase übergehen und somit die Rückbildung des Carbamats erfolgt. Auch war es nicht selbstverständlich, dass das aus der Reaktionszone zu isolierende Amin einfach abgetrennt werden konnte und nicht mit der Säure unter Bildung eines Säureamids abreagiert. Vielmehr kann das Amin vollständig und in sehr hoher Reinheit aus der Reaktionszone, die die Säure enthält, isoliert werden. Das erfindungsgemäße Verfahren lässt sich überraschenderweise auf eine große Zahl an Carbamaten anwenden, so dass sowohl aliphatische als auch aromatische Amine auf diese Weise hergestellt werden können. Auf diese Weise ist es möglich, ein Semibatch-Verfahren und in einer speziellen Ausführungsvariante auch ein kontinuierliches Verfahren zur Verfügung zu stellen. Das erfindungsgemäße Verfahren hat weiterhin den Vorteil, dass es ohne den Einsatz von Metallen und/oder Metallverbindungen verläuft.

Gegenstand der Erfindung ist somit ein Verfahren zur Herstellung von Aminen durch Spaltung von Carbamaten der Formel I 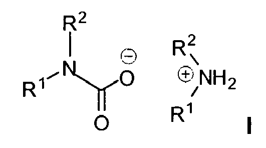 mit
R¹ = Wasserstoff, Alkyl, Aryl- oder Phenylgruppe,
R² = Alkyl, Aryl- oder Phenylgruppe,
wobei die Substituenten vom Typ R¹ und R² substituiert oder unsubstituiert sind,
das sich dadurch auszeichnet, dass die Spaltung des Carbamats in Gegenwart einer Säure der Formel II 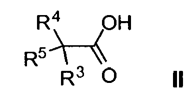 mit R³, R⁴ und R⁵ = Alkyl- oder Phenylgruppe,
wobei die Substituenten vom Typ R¹, R², R³, R⁴ und R⁵ gleich oder verschieden sind, durchgeführt wird.

Als Säuren werden in dem erfindungsgemäßen Verfahren Verbindungen gemäß der Formel II eingesetzt. Vorzugsweise werden in dem erfindungsgemäßen Verfahren sogenannte Neo- oder Kochsäuren eingesetzt. Diese Säuren haben den Vorteil, dass sie auf Grund des quartären α-Kohlenstoffatoms besonders hydrolyse- und oxidationsstabil sind. Bevorzugt werden Säuren gemäß der Formel II eingesetzt, die als Substituenten vom Typ R³, R⁴ und R⁵ eine Alkylgruppe aufweisen, insbesondere Säuren der Formel II mit einer Methylgruppe als Substituenten vom Typ R⁴ und R⁵ und als Substituenten vom Typ R³ eine Gruppe gemäß der Formel III

-(CH₂)ₙ-CH₃ III

mit n = 0 - 8, bevorzugt n = 2 - 7,
aufweisen.

Besonders bevorzugt werden in dem erfindungsgemäßen Verfahren hochsiedende Neosäuren, insbesondere mit einer Gesamtanzahl an Kohlenstoff von 5 bis 12. eingesetzt. Ganz besonders bevorzugt wird als Säure Neodecansäure (R⁴ und R⁵ = Methyl, R³ = -(CH₂)₅-CH₃) eingesetzt. In dem erfindungsgemäßen Verfahren können auch Mischungen dieser Säuren der Formel II eingesetzt werden, wobei diese Mischungen an Säuren auch Isomere der Säuren der Formel II aufweisen können.

Vorteilhaft ist der Einsatz von Säuren, die einen Siedepunkt von mindestens 150°C bei einem Druck von 1013 mbar aufweisen. Bevorzugt weisen die in dem erfindungsgemäßen Verfahren eingesetzten Säuren einen Siedepunkt von 160 bis 300 °C auf.

Die in dem erfindungsgemäßen Verfahren eingesetzten Säuren zeichnen sich zu einem durch ihren hohen Siedepunkt aus, aber auch durch ihre säurekatalytische Aktivität bei der Spaltung von Carbamaten.

In dem erfindungsgemäßen Verfahren werden Carbamate der Formel I in die entsprechenden Amine gespalten. Bevorzugt werden Carbamate der Formel I mit R¹ = Wasserstoff und R² = Alkylgruppe, besonders bevorzugt werden jedoch Carbamate der Formel I mit R¹ = Wasserstoff und R² = -(CH₂)ₙ-CH₃ mit n = 0 - 8, bevorzugt n = 2 - 7, in dem erfindungsgemäßen Verfahren eingesetzt. In einer besonderen Ausführungsform des erfindungsgemäßen Verfahrens werden Carbamate der Formel I mit R¹ = Wasserstoff und R² = Alkylgruppe, wobei die Alkylgruppe substituiert ist, eingesetzt. Insbesondere eignen sich für dieses Verfahren Carbamate der Formel I mit R¹ = Wasserstoff und R² = Alkylgruppe, die mit Aminogruppen, bevorzugt einer Aminogruppe, substituiert ist. So werden bevorzugt Carbamate der Formel I mit R¹ = Wasserstoff und R² = -(CH₂)ₙ-NH₂ eingesetzt mit n = 1 - 8, insbesondere 2 - 7.

Das zu spaltende Carbamat kann in dem erfindungsgemäßen Verfahren als Lösung, Suspension oder als Lösemittel-aufweisender Feststoff eingesetzt werden. Bevorzugt werden in dem erfindungsgemäßen Verfahren Lösemittel eingesetzt, die einen Siedepunkt von 30 bis 300°C, besonders bevorzugt von 50 bis 150°C, aufweisen.

Als Lösemittel für die Carbamate können in dem erfindungsgemäßen Verfahren Alkohole, wie beispielsweise Methanol, Ethanol, Isopropanol oder Cyclohexanol, aliphatische Kohlenwasserstoffe, wie beispielsweise Hexan, Heptan, Oktan, Nonan oder Decan, cycloaliphatische Kohlenwasserstoffe, wie beispielsweise Cyclohexan, oder aromatische Kohlenwasserstoffe, wie beispielsweise Toluol oder Xylol, eingesetzt werden. Die Konzentration an Carbamat in der eingesetzten Carbamat-Lösung oder auch Carbamat-Suspension beträgt vorzugsweise von 5 bis 80 Gew.-%, bevorzugt von 10 bis 50 Gew.-%.

Vorteilhaft ist es in dem erfindungsgemäßen Verfahren, die Säure bei der gewünschten Reaktionstemperatur in der Reaktionszone vorzulegen und erst anschließend das Carbamat der Reaktionszone zuzuführen.

Vorzugsweise wird an Säure in dem erfindungsgemäßen Verfahren von 0,1 bis 1000 Moleq., bevorzugt von 0,5 bis 500 Moleq. bezogen auf die eingesetzte Menge an Carbamat eingesetzt.

Durch den Einsatz von Säuren gemäß der Formel II ist eine entsprechend hohe Reaktionstemperatur bei der Zudosierung des Carbamats während der Spaltung in dem erfindungsgemäßen Verfahren möglich, wobei die Reaktionstemperatur ausreichend hoch ist für die Spaltung des Carbamats als auch ausreichend niedrig, so dass ein Übergang des Zielprodukts Amin in die Gas- und/oder Dampfphase unterbunden werden kann. Auf diese Weise kann das Lösemittel, welches durch die Zugabe Carbamats in die Reaktionszone eingetragen wird, rasch wieder aus der Reaktionszone entfernt werden. Die Spaltung des Carbamats in dem erfindungsgemäßen Verfahren wird vorzugsweise bei einer Reaktionstemperatur von 50 °C bis 320°C, bevorzugt von 120°C bis 250°C durchgeführt. Die Reaktionstemperatur wird vorzugsweise so gewählt, dass mit der Abtrennung des Kohlendioxids kein Zielprodukt Amin mitüberdestilliert oder mitgerissen werden kann. Im Allgemeinen ist eine Reaktionstemperatur im Sumpf vorteilhaft, die um 20 bis 30 K unterhalb der Siedetemperatur des Amins liegt.

Der Druck, bei dem die Spaltung des Carbamats in dem erfindungsgemäßen Verfahren durchgeführt wird, beträgt vorzugsweise von 20 mbar bis 2000 mbar, bevorzugt von 800 bis 1200 mbar und besonders bevorzugt von 950 bis 1100 mbar. Ganz besonders bevorzugt wird die Spaltung des Carbamats in dem erfindungsgemäßen Verfahren bei Atmosphärendruck durchgeführt.

Das erfindungsgemäße Verfahren kann sowohl als Batchprozess als auch als kontinuierlicher Prozess betrieben werden, bevorzugt wird es jedoch als kontinuierlicher Prozess betrieben.

In einer bevorzugten Ausführungsform des erfindungsgemäßen Verfahrens werden kontinuierliche Verweilzeitreaktoren, wie beispielsweise Rohrreaktoren, Fallfilm- oder Rieselfilmverdampfer, Dünnschichtverdampfer oder Dünnfilmextruder eingesetzt. Bei diesen Reaktortypen wird in dem erfindungsgemäßen Verfahren die Säure gemäß Formel II vorzugsweise im Kreislauf geführt und das zu spaltende Carbamat - beispielsweise in Form einer Lösung oder Suspension - in diese Vorrichtung der Reaktionszone zugeführt. Es ist auch möglich die Säure nicht im Kreislauf zu fahren und somit die Säure erst nach einer separaten Aufarbeitung dem Reaktor wieder zuzuführen oder zu verwerfen.

Bei der Spaltung entsteht Kohlendioxid, das somit zusammen mit dem in die Reaktionszone eingetragenen Lösemittel - beispielsweise Wasser- kontinuierlich aus der Reaktionszone entfernt werden kann. Aufgrund der starken CO₂-Entwicklung während der Spaltung des Carbamats ist es vorteilhaft, eine große Verdampferoberfläche zur Verfügung zu stellen, daher sind Fallfilm-, Riesel- oder Dünnschichtverdampfern für das erfindungsgemäße Verfahren besonders geeignet.

Damit das nach der Spaltung des Carbamats erhaltene Amin nicht mit dem ebenfalls entstehenden Kohlendioxid in Kontakt tritt und es somit zu einer Rückreaktion zum Carbamat kommt, wird das entstehende Kohlendioxid durch einen Inertgasstrom, beispielsweise Stickstoff, abgestrippt.

Im Anschluss an die Spaltung des Carbamats kann das aus der Spaltung gewonnene Amin in dem erfindungsgemäßen Verfahren abgezogen werden. Dies wird beispielsweise auf destillativem Wege durch weitere Erhöhung der Temperatur und/oder Absenkung des Druckes erreicht, wobei das Amin aus der Reaktionszone entfernt wird.

Die zurückbleibende Sumpfphase mit der Säure der Formel 11 kann in dem erfindungsgemäßen Verfahren anschließend erneut einer Spaltung von Carbamaten zur Verfügung gestellt werden, indem sich ein wiederholter Kreislauf oder im einfachsten Fall ein weiterer Semibatch-Zyklus, beispielsweise in einem Rührkesselreaktor, anschließt.

Das gemäß dem erfindungsgemäßen Verfahren gewonnene Amin hat im Allgemeinen eine so hohe Reinheit, dass keine weiteren Reinigungsschritte mehr erforderlich sind. Insbesondere können mittels dem erfindungsgemäßen Verfahren Amine mit einer GC-Reinheit > 98 Flächen-% erhalten werden. Sollte das Amin jedoch nicht in der gewünschten Reinheit vorliegen, so kann eine weitere Destillation oder Rektifikation sich anschließen.

Es ist vorteilhaft bei allen Aufarbeitungsschritten in dem erfindungsgemäßen Verfahren unter Inertgas zu arbeiten, um die erneute Bildung von Carbamaten, die sich aus dem Reaktionsprodukt Amin und CO₂ aus der Atmosphäre rückbilden können, zu verhindern.

Die nachfolgenden Beispiele sollen das erfindungsgemäße Verfahren zur Herstellung von Aminen näher erläutern, ohne dass die Erfindung auf diese Ausführungsform beschränkt sein soll.

### Beispiel 1 (erfindungsgemäß)

In einen 1 1 Kolben mit Rührer und einer 10 cm Füllkörperkolonne, einem Kolonnenkopf und einem Kühler werden 200 g Neodecansäure (Versatic10 der Firma Resolution) vorgelegt. Dieser Sumpf wird auf ca. 120°C erhitzt. Innerhalb von 2 Stunden werden 800 g einer 30 Gew.-%igen wässrigen Lösung eines Carbamats gemäß Formel I mit R¹ = H und R² = -(CH₂)₆-NH₂ (dies entspricht 0,87 mol an Carbamat) zudosiert. Innerhalb dieser 2 Stunden werden destillativ ca. 550 g Wasser entfernt und es werden ca. 19 1 Kohlendioxid frei. Die Ermittlung des entstehenden Kohlendioxids wurde mit einer geeigneten Gasuhr verfolgt. Nachdem die Kohlendioxidbildung beendet ist und das gesamte Wasser entfernt wurde, wird der Druck soweit reduziert, so dass auch das restliche Wasser entfernt werden kann. Unter weiterer Reduzierung des Druckes wird danach 1,6-Diaminohexan abdestilliert.

Es werden ca. 95 g an 99%igem 1,6-Diaminohexan gewonnen, dies entspricht einer Ausbeute von ca. 95 %.

### Vergleichsbeispiel 1

Das Vergleichsbeispiel 1 wird unter analogen Bedingungen wie in Beispiel 1 durchgeführt, allerdings werden statt 200 g Neodecansäure 200 g Marlotherm SH eingesetzt. Es wird keine thermische Spaltung des Carbamats beobachtet. Es konnte lediglich eine destillative Entfernung des Wassers beobachtet werden. Das nicht gespaltene Carbamat verbleibt im Sumpf.

### Vergleichsbeispiel 2

Das Vergleichsbeispiel 2 wird unter analogen Bedingungen wie in Vergleichsbeispiel 1 durchgeführt, allerdings wurde die Sumpftemperatur auf 200 °C erhitzt. Nun konnte eine thermische Carbamatspaltung beobachtet werden, wobei das entstehende Amin und Kohlendioxid gleichzeitig im Dampfraum des Reaktors vorliegen, so dass eine Rückreaktion des Carbamats beobachtet werden konnte.

In den Vergleichsbeispielen 1 und 2 konnte kein Amin isoliert werden.

### Beispiel 2 (erfindungsgemäß)

Ein Dünnschichtverdampfer aus Glas mit einer Oberfläche von 0,1 m² wird mit ca. 500 g/h Neodecansäure unter Zuhilfenahme einer Dosierpumpe beaufschlagt. Die Wärmeträgervorlauftemperatur am Dünnschichtverdampfer beträgt ca. 180 °C. Mit Hilfe einer zweiten Dosierpumpe wird der Dünnschichtverdampfer mit ca. 200 g/h einer 30 Gew.-%igen wässrigen Lösung eines Carbamats gemäß Formel I mit R¹ = H und R² = -(CH₂)₆-NH₂ beaufschlagt. Es destillieren ca. 120 g/h Wasser über Kopf ab. Gleichzeitig kann in Folge der CO₂-Abspaltung eine Gasentwicklung mit Hilfe einer üblichen Gasvolumenmessung verfolgt werden (es entstehen ca. 4-5 I/h Kohlendioxid). Im Sumpf des Dünnschichtverdampfers verbleiben Neodecansäure und 1,6-Diaminohexan, die anschließend durch Destillation entweder an einem weiteren Dünnschichtverdampfer oder durch einfache Kurzwegdestillation voneinander getrennt werden können.
Beide Verfahrensvarianten in Beispiel 2 führten zu einer Ausbeute an 1,6-Diaminohexan bezogen auf das eingesetzte Carbamat von ca. 95 %.

## Patentansprüche

1. Verfahren zur Herstellung von Aminen durch Spaltung von Carbamaten der Formel I 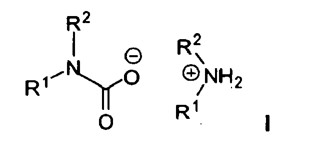 mit R¹ = Wasserstoff, Alkyl, Aryl- oder Phenylgruppe,
R² = Alkyl, Aryl- oder Phenylgruppe,
wobei die Substituenten vom Typ R¹ und R² substituiert oder unsubstituiert sind,
**dadurch gekennzeichnet,**
**dass** die Spaltung des Carbamats in Gegenwart einer Säure der Formel II 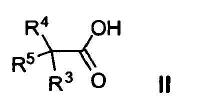 mit R³, R⁴ und R⁵ = Alkyl- oder Phenylgruppe,
wobei die Substituenten vom Typ R¹, R², R³, R⁴ und R⁵ gleich oder verschieden sind, durchgeführt und das entstehende Kohlendioxid durch einen Inertgasstrom abgestrippt wird.

2. Verfahren gemäß Anspruch 1,
**dadurch gekennzeichnet,**
**dass** eine Säure gemäß Formel II mit einer Methylgruppe als Substituenten vom Typ R⁴ und R⁵ und als Substituenten vom Typ R³ eine Gruppe gemäß der Formel III
-(CH₂)ₙ-CH₃ III
mit n=0-8
eingesetzt wird.

3. Verfahren gemäß Anspruch 1 oder 2 ,
**dadurch gekennzeichnet,**
**dass** als Säure Neodecansäure eingesetzt wird.

4. Verfahren gemäß zumindest einem der Ansprüche 1 bis 3,
**dadurch gekennzeichnet,**
**dass** das Carbamat als Lösung, Suspension oder als Lösemittel-aufweisender Feststoff eingesetzt wird.

5. Verfahren gemäß Anspruch 4,
**dadurch gekennzeichnet,**
**dass** das Lösemittel einen Siedepunkt von 30°C bis 300 °C aufweist.

6. Verfahren gemäß zumindest einem der Ansprüche 1 bis 5,
**dadurch gekennzeichnet,**
**dass** die Säure bei der gewünschten Reaktionstemperatur in der Reaktionszone vorgelegt und erst anschließend das Carbamat der Reaktionszone zugeführt wird.

7. Verfahren gemäß zumindest einem der Ansprüche 1 bis 6,
**dadurch gekennzeichnet,**
**dass** an Säure von 0,1 bis 1000 Moleq. bezogen auf die eingesetzte Menge an Carbamat eingesetzt wird.

8. Verfahren gemäß zumindest einem der Ansprüche 1 bis 7,
**dadurch gekennzeichnet,**
**dass** die Spaltung des Carbamats bei einer Reaktionstemperatur von 50 °C bis 320°C durchgeführt wird.

## Claims

1. Process for preparing amines by splitting carbamates of the formula I 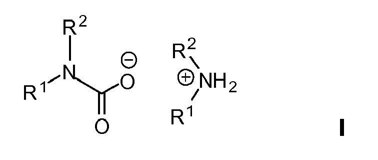 where R¹ = hydrogen, alkyl, aryl or phenyl group,
R² = alkyl, aryl or phenyl group,
where the substituents of the R¹ and R² type are substituted or unsubstituted,
**characterized in that**
the splitting of the carbamate is performed in the presence of an acid of the formula II 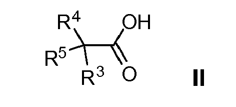 where R³, R⁴ and R⁵ = alkyl or phenyl group, where the substituents of the R¹, R², R³, R⁴ and R⁵ type are the same or different, and the carbon dioxide formed is stripped out by an inert gas stream.

2. Process according to Claim 1,
**characterized in that**
an acid of the formula II having a methyl group is used as the substituent of the R⁴ and R⁵ type and, as the substituent of the R³ type, a group of the formula III
**-(CH₂)ₙ-CH₃** **III**
where n = 0 - 8.

3. Process according to Claim 1 or 2,
**characterized in that**
the acid used is neodecanoic acid.

4. Process according to at least one of Claims 1 to 3,
**characterized in that**
the carbamate is used as a solution, suspension or as a solvent-comprising solid.

5. Process according to Claim 4,
**characterized in that**
the solvent has a boiling point of 30°C to 300°C.

6. Process according to at least one of Claims 1 to 5,
**characterized in that**
the acid is initially charged in the reaction zone at the desired reaction temperature and only then is the carbamate supplied to the reaction zone.

7. Process according to at least one of Claims 1 to 6,
**characterized in that**
0.1 to 1000 molar eq. of acid is used, based on the amount of carbamate used.

8. Process according to at least one of Claims 1 to 7,
**characterized in that**
the splitting of the carbamate is performed at a reaction temperature of 50°C to 320°C.

## Revendications

1. Procédé pour la préparation d'amines par coupure de carbamates de formule I 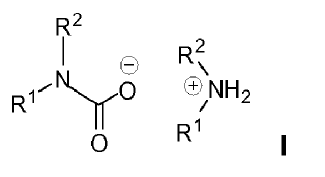 où R¹ = un atome d'hydrogène, un groupe alkyle, aryle ou phényle,
R² = un groupe alkyle, aryle ou phényle,
les substituants du type R¹ et R² étant substitués ou non substitués,
**caractérisé en ce qu'**on effectue la coupure du carbamate en présence d'un acide de formule II 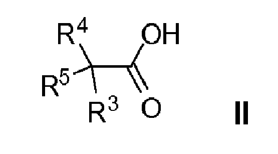 où R³, R⁴ et R⁵ = un groupe alkyle ou phényle,
les substituants du type R¹, R², R³, R⁴ et R⁵ étant identiques ou différents, et on élimine par entraînement par un courant de gaz inerte le dioxyde de carbone résultant.

2. Procédé selon la revendication 1, **caractérisé en ce qu'**on utilise un acide selon la formule II, comportant un groupe méthyle en tant que substituants du type R⁴ et R⁵ et, en tant que substituants du type R³, un groupe de formule III
-(CH₂)ₙ-CH₃ **III**
où n = 0-8.

3. Procédé selon la revendication 1 ou 2, **caractérisé en ce qu'**on utilise comme acide l'acide néodécanoïque.

4. Procédé selon au moins l'une des revendications 1 à 3, **caractérisé en ce que** le carbamate est utilisé sous forme de solution, suspension ou de solide comportant un solvant.

5. Procédé selon la revendication 4, **caractérisé en ce que** le solvant a un point d'ébullition de 30 °C à 300 °C.

6. Procédé selon au moins l'une des revendications 1 à 5, **caractérisé en ce qu'**on dispose au préalable l'acide, à la température de réaction désirée, dans la zone de réaction, et seulement ensuite on introduit le carbamate dans la zone de réaction.

7. Procédé selon au moins l'une des revendications 1 à 6, **caractérisé en ce qu'**on utilise de 0,1 à 1 000 équivalents molaires d'acide, par rapport à la quantité utilisée de carbamate.

8. Procédé selon au moins l'une des revendications 1 à 7, **caractérisé en ce que** la coupure du carbamate est effectuée à une température de réaction de 50 °C à 320 °C.
